# EUROPEAN PATENT SPECIFICATION

(11) **EP 3 162 455 B1**
(45) Date of publication and mention of the grant of the patent: **01.08.2018**
(21) Application number: 16195072.0
(22) Date of filing: 21.10.2016
(51) Int. Cl.: B09B 3/00, A61L 11/00, B09B 5/00, B29B 17/02, B29L 31/48

(54) **METHOD FOR PROCESSING A STARTING MATERIAL CONTAINING ORGANIC COMPONENTS.**
METHODE, UM EIN MATERIAL ZU BEARBEITEN, DAS ORGANISCHE BESTANDTEILE ENTHÄLT.
PROCÉDÉ DE TRAITEMENT D'UN MATÉRIAU CONTENANT DES COMPOSÉS ORGANIQUES.

(30) Priority: 26.10.2015 NL 2015658
(43) Date of publication of application: 03.05.2017
(73) Proprietor: Elsinga Beleidsplanning en Innovatie B.V., 3853 JA Ermelo (NL)
(72) Inventor: ELSINGA, Willem, 3853 JA ERMELO (NL); DUINDAM, Jelle Willem, 3853 JA ERMELO (NL)
(74) Representative: Algemeen Octrooi- en Merkenbureau B.V.

(56) References cited:
- EP-A1- 2 138 244
- EP-A1- 2 596 811
- EP-A2- 0 814 061
- GB-A- 1 117 549
- GB-A- 2 472 599
- US-A- 4 657 681

## Description

The present invention relates to a method for processing a starting material containing organic components which comprises one or more of diapers and incontinence materials, wherein the starting material is introduced into an autoclave and treated under high pressure and high temperature conditions to obtain one or more residual flows. The present invention further relates to the use of an autoclave for processing a starting material containing organic components.

In the Netherlands and Europe a strong emphasis is placed on waste prevention, reuse of raw materials from the waste flows that are still being produced and energy recovery. By conducting a waste policy aimed at prevention and reuse, Europe will be less dependent on the import of raw materials and energy. At the same time, the related burden on the environment is reduced. Dutch and European policies are well-attuned and integrated by aligning national legislation with European regulations.

By now, separation of household waste has become an important instrument in Europe, and certainly in the Netherlands, in this regard, with the various partial waste flows in some cases being obtained at the source and in other cases being obtained separately through subsequent separation and being reused. This policy is very successful and leads to the separation and reuse of plastics, glass, metals, paper, organic waste, inert fractions such as stone and rubble, and a residual fraction that is to be incinerated yet in a waste energy plant. Apart from the residual fraction, all the other separated partial flows are reused. Thus, organic waste is subjected to a fermentation and recomposting process, resulting in biogas as a renewable energy source and reusable compost. Plastics are separated and post-separated and reprocessed into granulate for the production of new plastic articles. In families with young children, for example, diapers can constitute as much as 50% of the weight and volume of the household waste.

For a specific starting material containing organic components, such as diapers and incontinence material, a number of routes exist for processing such materials.

Possible routes for processing diaper waste currently include:
- the waste incineration plant route: in this route the diapers are collected as part of the residual waste and incinerated in an incineration plant;
- the composting route: in this route the diapers are collected as part of the organic waste and processed in a composting plant;
- the fermentation route: in this route the diapers are collected as part of the organic waste and processed in a fermentation plant;
- the separated collecting route: in this route the diapers are collected separately and separated into pulp, plastic and other fractions in a separation plant.

In the case of diapers, there are a number of components of which the diapers are composed, such as super-absorbent components (SAP), plastics such as polyethylene and polypropylene, but also pulp components. Research has shown that the composition of diapers comprises 60% dry matter pulp and 16% plastic. The pulp content has decreased to 40% in the course of time, about 30% of which is CTMP pulp, which is difficult to decompose in composting and fermentation plants. This means that a relatively small part of the diaper can be decomposed in composting and fermentation plants. In addition to that, super-absorbent polymers are considered to be less biodegradable. This means that more than 60% of the amount of dry matter of the diaper remains unaffected by the composting and fermentation process. Furthermore, diapers and in particular incontinence materials may contain pathogens and (residue of) medicines. For the reuse of residual flows that remain after the treatment of diapers and incontinence materials it is important that pathogens be killed and medicines be removed. This is highly doubtful especially if these materials are composted or fermented along with the existing organic waste flows.

Methods for separating materials that contain such super-absorbent components are known from, inter-alia, European patent publication EP 2 782 651. According to the method that is known therefrom, the starting material, which contains super absorbent polymer, a fibre and a plastic, is first reduced, after which a salt is added to the reduced material. The material, which is said not to comprise slurry or form part of a slurry, is then agitated. It is desirable that the material be heated by adding steam during the aforesaid treatment, using aluminium sulphate as a specific salt.

Japanese publication JP 2012-080825 discloses a device and a method for producing pet food, using birds and beasts as starting materials. The device for producing pet food that is used therein comprises a pressure container provided with a stirring mechanism comprising a rotary shaft, wherein a plurality of stairs of wings are rotatably attached to the rotary shaft at fixed intervals.

From International application WO 2006/006606 there is known a process for producing fuel, in which waste with a high water content, for example organic waste, animal excrements, fish waste, can be treated within a short cycle time so as to realise a conversion into a form suitable for use as a fuel. The process comprises injecting high-pressure steam into a waste-containing vessel provided with an agitation mechanism, in which a pressure of at least 1.96 MPa prevails, and increasing the temperature thereof. The injection of high-pressure steam is discontinued when the temperature of the material section in a lower part of the vessel corresponds to the temperature of the vacant zone in an upper part thereof.

International application WO 2015/097254 discloses a method for the continuous thermal hydrolysis of sludge, said method comprising at least: a. a step of destructuring the sludge; b. a step of thermal hydrolysis of the thus destructured sludge in a thermal hydrolysis reactor; and c. a step of cooling the hydrolysed sludge. According to WO 2015/097254, the destructuring step comprises a step of introducing the sludge to be treated into a dynamic mixer and subsequently heating the sludge from said dynamic mixer.

EP 0 814 061 relates to a method for hydrothermally treating an aqueous medium containing an organic matter, comprising the steps of: pretreating the aqueous medium without adding an oxidizing agent at a first temperature ranging from 200°C to 374°C under a pressure of not less than a vapor pressure of the aqueous medium at the first temperature; and treating the aqueous medium with an oxidizing agent in an amount sufficient for decreasing a chemical oxygen demand of the aqueous medium at a second temperature ranging from 200°C to 374°C under a pressure of not less than a vapor pressure of tile aqueous medium at the second temperature. Examples of organic matter are municipal waste, night soil, sewage sludge, industrial wastes.

EP 2 138 244 relates to a method for biogenic waste treatment, comprising the following steps: a first step wherein the biogenic waste is evenly mixed with water vapor so that the temperature reaches 130-190°C and the pressure reaches 1.5-1.9 MPa after being mixed, and this state is maintained for 1-25 minutes, a second step wherein the pressure of the mixture of said biogenic waste and water vapor is reduced to normal pressure so as to conducting low-molecularization of the biogenic waste and obtain a low-molecularized mixture, and a third step wherein the low-molecularized mixture is separated so as to obtain the final product.

Korean publication KR 101348489 discloses a method for treating sludge using high-frequency dielectric heating.

From International application WO 2013/171248 there is furthermore known a method for separating mixed cellulose-plastic products, wherein the mixed cellulose-plastic product is mixed with water and subsequently introduced into a stirred autoclave. The material present in the autoclave is heated during continued stirring at a rotational speed of 50 to 350 min-1 to a temperature of 150 °C to 250 °C at a pressure of 1.2 to 3.0 MPa. Maintaining the aforesaid conditions for a period of 0.3 to 6 hours, the cellulose present in the starting material is hydrolysed, with the plastics agglomerating into packages of spherical shapes.

The method known from the aforesaid International application is conditional upon water being added to the starting material. The addition of a supplementary amount of water, however, leads to an increased energy requirement of the process. Moreover, the end product obtained with the aforesaid method will contain a large amount of water, which is undesirable. In addition to that, the end product obtained with the aforesaid method is one large plastic sphere, which plastic sphere has the drawback of being difficult to remove from the autoclave. This means that the autoclave must be provided with a large outlet opening in order to prevent the autoclave from becoming obstructed upon being discharged. In the case of a high-pressure autoclave, the consequence of this is that a costly and technically complex measure must be taken, which is undesirable in practice. In addition, such plastic spheres are very hot after the process, with cooling down taking place at a very slow rate.

The object of the present invention is therefore to provide a method for processing a starting material containing organic components which comprises one or more of diapers and incontinence materials, wherein one or more residual flows are obtained, which can be reused in a simple and useful manner.

Another object of the present invention is to provide a method for processing a starting material containing organic components which comprises one or more of diapers and incontinence materials, wherein the formation of a large plastic sphere is prevented.

Yet another object of the present invention is to provide a method for processing a starting material containing organic components which comprises one or more of diapers and incontinence materials, wherein use is made of an energetically optimally advantageous process.

Yet another object of the present invention is to provide a method for processing a starting material containing organic components which comprises one or more of diapers and incontinence materials, wherein the medicines present in such materials are decomposed.

The present invention thus relates to a method for processing a starting material containing organic components which comprises one or more of diapers and incontinence materials, wherein the starting material is introduced into an autoclave and treated under high temperature conditions so as to obtain one or more residual flows, wherein the method comprises the following steps:
i) transferring the aforesaid starting material containing organic components to an autoclave provided with a stirring mechanism,
ii) heating the aforesaid starting material containing organic components in the aforesaid autoclave while driving the stirring mechanism at a speed in the range of 5-50 rpm, using a temperature of at least 200 °C and a pressure of at least 20 bar,
iii) maintaining the conditions of step ii) for a period of at least 0.1 hour,
iv) removing the one or more residual flows thus formed from the aforesaid autoclave.

One or more of the above objects are achieved using the aforesaid steps i) - iv).

The present inventors have in particular found that if an autoclave whose stirring mechanism is provided with one or more elements that rotate along the inner wall of the autoclave is used in step i), the formation of a large plastic sphere is prevented. It is thus desirable not to use a stirring mechanism that consists of a centrally positioned small mixing auger that rotates at a high speed but a stirring mechanism with arms which are long and curved and/or which are arranged at an angle to the horizontal. Such long, curved arms are driven at a low speed, with the curved arms being moved along the walls of the autoclave, so that a good internal mixing in the autoclave is realised.

The phrase "along the walls of the autoclave" is understood to mean that the spacing between the end of the stirring mechanism and the wall of the autoclave is at most 10 cm, preferably at most 5 cm, particularly preferably at most 1 cm. In a special embodiment, such a stirring mechanism may comprise one or more additional elements besides the aforesaid curved arms, which elements are spaced from the inner wall of the autoclave by a distance greater than the aforesaid distances.

The inventors have found that, using such a stirring mechanism, a multitude of small plastic agglomerates are formed, after which the contents of the autoclave can subsequently be removed in a simple and advantageous manner. If, on the other hand, a centrally positioned small stirring mechanism is used, a central vortex is created, but in the present method said central vortex is prevented, so that the formation of a large plastic sphere is prevented. According to the present method, a multitude of small plastic agglomerates are thus formed in the autoclave if use is made of such a stirring mechanism. Such small plastic agglomerates can subsequently be removed from the autoclave in a simple manner upon completion of the present method. It is desirable that step iv) be carried out after the temperature of the autoclave has decreased to preferably at most 110 °C. The present inventors have also found that, in addition to the special use of the aforesaid stirring mechanism, a special use of specific mixing ratios of diapers and incontinence materials on the one hand and other organic components on the other hand is desirable in order to obtain small agglomerates of plastic material.

An advantageous effect of the stirring mechanism that is preferably used in the present invention is that it is capable of mixing the material in the autoclave well by means of the arms rotating along the wall. The material is turned over by the stirring arms, as it were. This type of stirring mechanism also functions well if the contents of the reactor change into high viscosity liquids during the standing time. Other stirring mechanism, such as auger-like centrally disposed high rpm stirring mechanisms (so-called "axial flow marine impellers") are unable to perform this function. Such stirring mechanisms would rotate in a void, the material being flung away, within a few seconds, and the material would not be mixed in this way.

Mixing by means of the stirring mechanism that is preferably used in the present invention leads to a quick and homogeneous heating of the material while steam is being added, because the steam is distributed throughout the material. This is desirable in order to ensure a uniform heat treatment and obtain a uniform treatment of the entire contents. In this way, overtreatment of some parts of the contents, as it were, is prevented. Upon fermentation of the resulting slurry in a downstream fermenter, the method according to the present invention provides a strongly increased gas yield, but in the case of overtreatment the gas yield will decrease again, as it has been found that in a downstream fermenter the material will be more difficult for bacteria to convert.

Yet another advantageous aspect of the stirring mechanism that is preferably used in the present invention relates to the manner in which the starting material to be treated is supplied. By their very nature, diapers are not very heavy and they are often supplied in plastic bags, so that a lot of air is enclosed. As a consequence of this, only a limited weight of diapers fits in the volume of the autoclave and only few kilograms of material can be processed with each batch. By rotating the stirring mechanism in opposite direction for a short period during or after filling, the material is not pushed up but down into the reactor and thus compressed. This has the advantage that in this way up to 35% more material will fit in the reactor, resulting in an enormous capacity increase.

Another advantageous aspect of the stirring mechanism that is preferably used in the present invention is that it is capable of compressing and mixing solid materials such as diapers and solidified wastewater treatment plant sludge in an advantageous manner and effecting a uniform temperature treatment while steam is being added.

Although mention is made of diapers and incontinence material in the present description, it should be understood that the starting material containing organic components that is to be treated may also comprise other, inevitable components. Think in this context of tissues, moist toilet paper, wet wipes, sanitary napkins, toilet paper and the like . In many municipalities special bags are distributed for collecting such waste materials therein. Diapers and incontinence materials are thus collected separately via special collecting bags which are usually available free of charge. The separate collection enables the municipality to recycle such materials.

The present method is further characterised in that a pressure of at least 30 bar, preferably at least 35 bar, particularly preferably 40-45 bar, is used in step ii). The result of using such a pressure is that the reactions in the autoclave will proceed in an advantageous and quick manner.

It is furthermore preferable that a temperature of at least 220 °C, preferably at least 250 °C, is used in step ii). Such a temperature is in particular advantageous for converting the starting material containing organic components into one or more residual flows, in particular obtaining residual flows which are not formed as one large lump in the autoclave. Such a temperature can for example be realised by injecting steam into the autoclave. As a result of the high temperature, the present process will proceed much more quickly and medicines will decompose more quickly. As a result, the cycle time of a charge will be shorter and the capacity of the plant will increase.

To effect a good mixing in the autoclave it is desirable that a stirring mechanism having a speed in a range of 10 - 40 rpm, preferably 20 - 40 rpm, in particular 10-30 rpm be used in step ii). The present inventors have found that, using such a stirrer rpm, the contents of the autoclave are turned over, as it were. In this way a different flow pattern rather than one vortex is created, so that the formation of one large agglomerate is prevented and a multitude of small agglomerates are formed. As a result of the fact that such small agglomerates are formed, said agglomerates can be easily discharged from the autoclave along with the slurry, so that the autoclave may be provided with a small outlet opening. Such a small outlet opening is technically easy to realise in a high-pressure autoclave. In addition, the use of such a stirrer rpm causes the temperature to be evenly distributed over the contents of the autoclave, in particular when the present method is started, and the formation of so-called cold and hot spots is prevented. The use of such a stirrer rpm prevents the formation of a large vortex in which a large plastic sphere would form. The low rpm is conducive to the formation of small agglomerates throughout the material, so that the reactor can be configured with a small outlet opening and clogging by large chunks of plastics is prevented. The small agglomerates also have the advantage that cooling, washing and drying is much easier than is the case with large chunks of plastics with a hot core and entrapped dirt.

It is preferable that step iii) is carried out for at most 0.3 hours. The use of the aforesaid process conditions makes it possible to use such a short cycle time.

The present inventors have found that, using the aforesaid process conditions, a quick hydrolysis of the cellulose components takes place, so that a relatively short residence time in the autoclave is possible. Because the residence time in the autoclave is significantly shorter than, for example, in the above-discussed International application WO 2013/171248, the capacity of the autoclave will be increased as well whilst maintaining the intended functionality, wherein in particular the inactivation of medicines and the killing off of pathogenic components must be considered in the case of diapers. Thus it is desirable that the one or more residual flows obtained after step iv) contain a percentage of active ingredients of medicines which is lower than the percentage of active ingredients of medicines of the starting material containing organic components that was supplied to the autoclave in step i).

It has also been found that, using the present method, the residual flows obtained therewith can advantageously be reused. The residual flows obtained in the processing of, for example, diapers and incontinence materials can be reused, in which regard the present method makes a useful contribution to the environment.

The present method is in particular suitable if in addition to one or more of diapers and incontinence materials also other additional components, preferably in the proportions to be mentioned below, are simultaneously processed in the autoclave, in particular one or more additional components selected from the group of sludge, in particular sludge from a wastewater purification plant, organic waste digestate and emulsifiers.

If also other additional components besides one or more of diapers and incontinence materials are simultaneously processed in the autoclave, it is preferable that the amount of additional components in the aforesaid starting material containing organic components is 10-50 wt.%, based on the weight of the total starting material. According to another embodiment, the amount of additional components in the aforesaid starting material containing organic components is 20-40 wt.%, based on the weight of the total starting material.

The present inventors have further found that also sludge from a water purification plant can be used as a starting material containing organic components. The present inventors have found that the use of certain mixtures, for example wastewater purification plant sludge and diapers, in the aforesaid mixing ratios can lead to a particular behaviour of the plastics by which small plastic spheres are formed. An advantage is that the reactor can be configured to have a small outlet opening and that no obstructions will take place during removal of the contents.

To realise a method that is advantageous from an energy point of view it is desirable that no additional amount of water be added to the autoclave in step i). The stirring mechanism that is preferably used in the present methods renders unnecessary the addition of water for improving the mixability of the starting material and the distribution of heat in the autoclave. By omitting to add an additional amount of water, less material needs to be heated, so that energy is saved.

It is desirable that a separation step be carried out on the one or more residual flows obtained after step iv) so as to obtain a residual flow that is rich in plastic and an organic slurry, wherein said organic slurry can additionally be separated into a high-fibre fraction and an aqueous fraction. The organic slurry, or in particular the aqueous fraction separated therefrom, can be supplied to a fermentation plant. The high-fibre fraction can be used as a starting material, for example for reclaiming fibres therefrom for the production of paper and comparable products.

The present invention further relates to the use of an autoclave provided with a stirring mechanism to perform the method for processing a starting material containing organic components which comprises one or more of diapers and incontinence materials into one or more residual flows, wherein an autoclave is used whose stirring mechanism is provided with one or more elements that rotate along the inner wall of the autoclave.

The present invention will now be explained by means of a number of examples, in which connection it should be noted, however, that the present invention is by no means limited to such special examples.

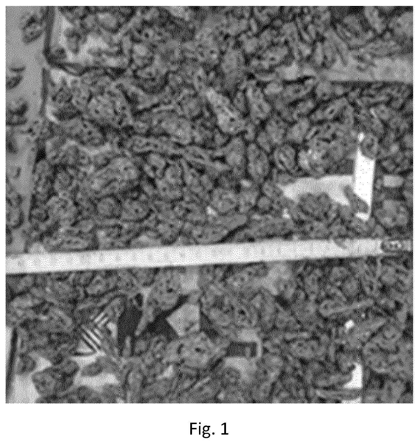

The appended figure is a photographic representation of small agglomerates.

### Example 1

An amount of starting material consisting of used baby diapers and sludge, viz 70 wt.% of diapers and 30 wt.% of wastewater purification plant sludge (solidified: dry matter content: 25%) was introduced into a reactor vessel with a volume of 300 I (fill ratio 66%, bulk density 500 kg/m³) and heated under pressure while stirring took place using a stirring mechanism positioned centrally in the reactor. The stirring mechanism (type of stirring mechanism: curved arms moving along the wall) was driven at a speed of 19 rpm. The following cycle time was used: 10 minutes heating, 10 minutes standing time, 20 minutes cooling, 5 minutes discharging and 10 minutes filling for a next batch. Temperature and pressure during standing time: temperature between 250 °C and 255 °C, pressure between 40 and 43 bar saturated steam. The treatment was stopped by releasing the pressure and cooling the contents. It appeared to be possible to carry out 5 cycles every 8 hours.

It was found that a complete separation of the plastic had taken place and that the other parts of the diapers had dissolved in the form of a homogeneous slurry. The plastic was floating in the slurry in the form of small agglomerates having a weight corresponding to 11% of the treated amount of diapers. In the appended figure a photographic representation of such small agglomerates is shown.

### Example 2

In the next test, a starting material exclusively comprising diapers was again introduced into a reactor vessel (fill ratio 66%, 500 kg/m³) and heated to a temperature of 258 °C at a pressure of 43 bar. The reactor was stirred using a stirring mechanism. The stirring mechanism was positioned at the bottom of the reactor vessel, mounted on the end of a stirring shaft. The stirring mechanism consisted of a metal part disposed transversely to the stirring shaft, with two upward-sloping stirring blades at the two ends, which moved along the inner side of the upright wall of the reactor vessel, spaced therefrom by a few millimetres. The stirring mechanism rotated at a speed of 25 rpm. After 10 minutes the treatment was stopped by cooling the contents of the reactor vessel and releasing the pressure. The reactor was opened and subsequently discharged. It could be clearly seen in the reactor that the plastic had separated both in the form of a floating cake and in the form of small agglomerates. The remainder of the diapers had dissolved into a homogeneous slurry.

### Example 3

In the reactor vessel as used in example 1, a certain amount of medicines, in particular sulphamethoxazole (antibiotic), Estradiol (contraceptive), Ibuprofen (painkiller), diclofenac (painkiller) and carbamezapine (anti-epilectic) was added to the starting material to be processed. The medicines were used in the form as sold in pharmacies, so that the starting concentrations of the active ingredients in the starting materials to be processed in the autoclave were precisely known. To obtain a good dissolvability of said medicines in the sample to be treated containing the mixture of medicines, the medicines were reduced in advance, dissolved in water and subsequently introduced into the autoclave. The process conditions in example 3 were identical to those described in example 1. After completion of the process, the concentrations of the medicines were measured again. The results showed that the treatment in the autoclave had resulted in a significant reduction of the percentages of active ingredients.

From the above-discussed examples it is apparent that the special process that is used results in a residual flow that can advantageously be further processed (see example 2). The composition of the residual flow thus obtained can be further improved if an additional component, for example sludge, is processed in the autoclave along with the starting material containing organic components (diapers in the case of example 1). The residual flow obtained therewith can be regarded as a slurry in the form of small agglomerates, wherein the formation of a floating cake is minimised. The inventors have further shown in example 3 that the special process that is used makes it possible to decompose medicines to a significant degree.

## Claims

1. A method for processing a starting material containing organic components which comprises one or more of diapers and incontinence materials, wherein the starting material is introduced into autoclave and treated under high pressure and high temperature conditions to obtain one or more residual flows, **characterised in that** the method comprises the following steps:
i) transferring the aforesaid starting material containing organic components to an autoclave provided with a stirring mechanism provided with one or more elements that rotate along the inner wall of the autoclave,
ii) heating the aforesaid starting material containing organic components in the aforesaid autoclave while driving the stirring mechanism at a speed in the range of 5-50 rpm, using a temperature of at least 200 °C and a pressure of at least 20 bar,
iii) maintaining the conditions of step ii) for a period of at least 0.1 hour,
iv) removing the one or more residual flows thus formed from the aforesaid autoclave.

2. A method according to claim 1, **characterised in that** in said autoclave as used step i) the spacing between the ends of said one or more elements and said inner wall is preferably at most 10 cm, in particular at most 5 cm, especially at most 1 cm.

3. A method according to any one of the preceding claims, **characterised in that** a pressure of at least 30 bar, preferably at least 35 bar, particularly preferably 40-45 bar, is used in step ii).

4. A method according to any one of the preceding claims, **characterised in that** a temperature of at least 220 °C, preferably at least 250 °C, is used in step ii).

5. A method according to any one of the preceding claims, **characterised in that** for said stirring mechanism a speed in a range of 10 - 40 rpm, preferably 20 - 40 rpm, particularly preferably 10-30 rpm is used in step ii).

6. A method according to any one of the preceding claims, **characterised in that** step iii) is carried out for at most 0.3 hours.

7. A method according to any one of the preceding claims, **characterised in that** said starting material containing organic components comprises one or more additional components selected from the group of sludge, in particular sludge from a wastewater purification plant, organic waste digestate and emulsifiers.

8. A method according to claim 7, **characterised in that** the amount of additional components in the aforesaid starting material containing organic components is 10-50 wt.%, preferably 20-40 wt.%, based on the weight of the total starting material.

9. A method according to any one of the preceding claims, **characterised in that** no additional amount of water is added to the autoclave in step i).

10. A method according to any one of the preceding claims, **characterised in that** a separation step is carried out on the one or more residual flows obtained after step iv) so as to obtain a residual flow that is rich in plastic and an organic slurry, wherein preferably said organic slurry is additionally separated into a high-fibre fraction and an aqueous fraction.

11. A method according to claim 10, **characterised in that** the organic slurry, in particular the aqueous fraction separated therefrom, is supplied to a fermentation plant.

12. A method according to any one of the preceding claims, **characterised in that** step iv) is carried out after the temperature of the autoclave has decreased to at most 110 °C.

13. A method according to claim 10, **characterised in that** the plastics are separated from the aforesaid residual flow that is rich in plastics and reprocessed into a material flow that is suitable for being reused.

14. A method according to any one of the preceding claims, **characterised in that** the one or more residual flows obtained after step iv) contain a percentage of active ingredients of medicines which is lower than the percentage of active ingredients of medicines of the starting material containing organic components that was supplied to the autoclave in step i).

15. Use of an autoclave provided with a stirring mechanism to perform the method according to any one of claims 1 to 14 for processing a starting material containing organic components which comprises one or more of diapers and incontinence materials into one or more residual flows, wherein the stirring mechanism is provided with one or more elements that rotate along the inner wall of the autoclave, said stirring mechanism being provided with arms which are long and curved and/or which are arranged at an angle to the horizontal, wherein the spacing between the ends of said one or more elements and said inner wall is at most 10 cm.

## Patentansprüche

1. Verfahren zum Verarbeiten eines organische Komponenten enthaltenden Ausgangsmaterials, das eine oder mehrere Windeln und/oder ein oder mehrere Inkontinenzmaterialien umfasst, wobei das Ausgangsmaterial in einen Autoklav eingeführt und unter Hochdruck- und Hochtemperaturbedingungen behandelt wird, um einen oder mehrere Restflüsse zu erhalten,
**dadurch gekennzeichnet, dass**
das Verfahren die folgenden Schritte umfasst:
i) Überführen des zuvor genannten organische Komponenten enthaltenden Ausgangsmaterials in einen Autoklav, der mit einem Rührmechanismus versehen ist, der mit einem oder mehreren Elementen, die entlang der inneren Wand des Autoklavs rotieren, versehen ist,
ii) Heizen des zuvor genannten organische Komponenten enthaltenden Ausgangsmaterials in dem zuvor genannten Autoklav während des Betreibens des Rührmechanismus bei einer Geschwindigkeit im Bereich von 5 U/min bis 50 U/min unter Verwendung einer Temperatur von wenigstens 200 °C und eines Drucks von wenigstens 20 bar,
iii) Aufrechterhalten der Bedingungen aus Schritt ii) für eine Zeitspanne von wenigstens 0,1 Stunden,
iv) Entfernen des einen oder der mehreren somit gebildeten Restflüsse aus dem zuvor genannten Autoklav.

2. Verfahren nach Anspruch 1,
**dadurch gekennzeichnet, dass**
in dem genannten, wie in Schritt i) verwendeten, Autoklav der Abstand zwischen den Enden des genannten einen oder der genannten mehreren Elemente und der genannten inneren Wand vorzugsweise höchstens 10 cm, insbesondere höchstens 5 cm, weiter insbesondere höchstens 1 cm, beträgt.

3. Verfahren nach einem der vorherigen Ansprüche,
**dadurch gekennzeichnet, dass**
ein Druck von wenigstens 30 bar, vorzugsweise wenigstens 35 bar, insbesondere vorzugsweise 40 bar bis 45 bar, in Schritt ii) verwendet wird.

4. Verfahren nach einem der vorherigen Ansprüche,
**dadurch gekennzeichnet, dass**
eine Temperatur von wenigstens 220 °C, vorzugsweise wenigstens 250 °C, in Schritt ii) verwendet wird.

5. Verfahren nach einem der vorherigen Ansprüche,
**dadurch gekennzeichnet, dass**
für den genannten Rührmechanismus eine Geschwindigkeit in einem Bereich von 10 U/min bis 40 U/min, vorzugsweise 20 U/min bis 40 U/min, insbesondere vorzugsweise 10 U/min bis 30 U/min, in Schritt ii) verwendet wird.

6. Verfahren nach einem der vorherigen Ansprüche,
**dadurch gekennzeichnet, dass**
Schritt iii) höchstens für 0,3 Stunden ausgeführt wird.

7. Verfahren nach einem der vorherigen Ansprüche,
**dadurch gekennzeichnet, dass**
das genannte organische Komponenten enthaltende Ausgangsmaterial ein oder mehrere zusätzliche aus der Gruppe von Schlamm, insbesondere Schlamm aus einer Abwasserreinigungsanlage, organischem Abfallgärgut und Emulgatoren ausgewählte Komponenten enthält.

8. Verfahren nach Anspruch 7,
**dadurch gekennzeichnet, dass**
der Anteil von zusätzlichen Komponenten in dem zuvor genannten organische Komponenten enthaltenden Ausgangsmaterial basierend auf dem Gewicht des gesamten Ausgangsmaterials 10 Gew.-% bis 50 Gew.-%, vorzugsweise 20 Gew.-% bis 40 Gew.-%, beträgt.

9. Verfahren nach einem der vorherigen Ansprüche,
**dadurch gekennzeichnet, dass**
dem Autoklav in Schritt i) keine zusätzliche Menge an Wasser hinzugefügt wird.

10. Verfahren nach einem der vorherigen Ansprüche,
**dadurch gekennzeichnet, dass**
bei dem einen oder den mehreren nach Schritt iv) erhaltenen Restflüssen ein Trennschritt durchgeführt wird, um einen Restfluss, der reich an Kunststoff ist, und einen organischen Schlamm zu erhalten, wobei, vorzugsweise, der genannte organische Schlamm zusätzlich in einen faserreichen Teil und einen wässrigen Teil getrennt wird.

11. Verfahren nach Anspruch 10,
**dadurch gekennzeichnet, dass**
der organische Schlamm, insbesondere der davon getrennte wässrige Teil, einer Fermentationsanlage zugeführt wird.

12. Verfahren nach einem der vorherigen Ansprüche,
**dadurch gekennzeichnet, dass**
Schritt iv) durchgeführt wird, nachdem die Temperatur des Autoklavs auf höchstens 110 °C gesunken ist.

13. Verfahren nach Anspruch 10,
**dadurch gekennzeichnet, dass**
die Kunststoffe von dem zuvor genannten Restfluss, der reich an Kunststoffen ist, getrennt und in einen Materialfluss, der geeignet ist, wieder verwendet zu werden, wiederaufbereitet werden.

14. Verfahren nach einem der vorherigen Ansprüche,
**dadurch gekennzeichnet, dass**
der eine oder die mehreren nach Schritt iv) erhaltenen Restflüsse einen Anteil an aktiven Ingredienzen von Arzneimitteln enthalten, der niedriger ist als der Anteil an aktiven Ingredienzen von Arzneimitteln des organische Komponenten enthaltenden Ausgangsmaterials, das dem Autoklav in Schritt i) zugeführt wurde.

15. Verwendung eines mit einem Rührmechanismus versehenen Autoklavs, um das Verfahren nach einem der Ansprüche 1 bis 14 zum Verarbeiten eines organische Komponenten enthaltenden Ausgangsmaterials, das eine oder mehrere Windeln und/oder ein oder mehrere Inkontinenzmaterialien umfasst, in einen oder mehrere Restflüsse durchzuführen, wobei der Rührmechanismus mit einem oder mehreren Elementen, die entlang der inneren Wand des Autoklavs rotieren, versehen ist, wobei der genannte Rührmechanismus mit Armen, die lang und gebogen sind und/oder die in einem Winkel zur Horizontalen angeordnet sind, versehen ist, wobei der Abstand zwischen den Enden des genannten einen oder der genannten mehreren Elemente und der genannten inneren Wand höchstens 10 cm beträgt.

## Revendications

1. Procédé de traitement d'une matière première contenant des composants organiques qui comprend un(e) ou plusieurs parmi des couches et des matériels d'incontinence, dans lequel la matière première est introduite dans un autoclave et traitée dans des conditions de haute pression et de température élevée pour obtenir un ou plusieurs flux résiduel(s), **caractérisé en ce que** le procédé comprend les étapes suivantes consistant :
i) à transférer la matière première susmentionnée contenant des composants organiques à un autoclave pourvu d'un mécanisme d'agitation doté d'un ou de plusieurs élément(s) qui tourne/tournent le long de la paroi interne de l'autoclave,
ii) à chauffer la matière première susmentionnée contenant des composants organiques dans l'autoclave susmentionné tout en entraînant le mécanisme d'agitation à une vitesse située dans la plage allant de 5 à 50 tr/min, en utilisant une température d'au moins 200°C et une pression d'au moins 20 bars,
iii) à maintenir les conditions de l'étape ii) pendant une période d'au moins 0,1 heure,
iv) à éliminer le ou les plusieurs flux résiduel(s) ainsi formé(s) de l'autoclave susmentionné.

2. Procédé selon la revendication 1, **caractérisé en ce que** dans ledit autoclave tel qu'utilisé dans l'étape i) l'espacement entre les extrémités desdits un ou plusieurs élément(s) et ladite paroi interne est de préférence d'au plus 10 cm, en particulier d'au plus 5 cm, notamment d'au plus 1 cm.

3. Procédé selon l'une quelconque des revendications précédentes, **caractérisé en ce qu'**une pression d'au moins 30 bars, de préférence d'au moins 35 bars, de manière particulièrement préférée de 40 à 45 bars, est utilisée dans l'étape ii).

4. Procédé selon l'une quelconque des revendications précédentes, **caractérisé en ce qu'**une température d'au moins 220°C, de préférence d'au moins 250°C, est utilisée dans l'étape ii).

5. Procédé selon l'une quelconque des revendications précédentes, **caractérisé en ce que** pour ledit mécanisme d'agitation, une vitesse située dans une plage allant de 10 à 40 tr/min, de préférence de 20 à 40 tr/min, de manière particulièrement préférée de 10 à 30 tr/min, est utilisée dans l'étape ii).

6. Procédé selon l'une quelconque des revendications précédentes, **caractérisé en ce que** l'étape iii) est réalisée pendant au plus 0,3 heure.

7. Procédé selon l'une quelconque des revendications précédentes, **caractérisé en ce que** ladite matière première contenant des composants organiques comprend un ou plusieurs composant(s) supplémentaire(s) choisi(s) dans le groupe constitué par des boues, en particulier les boues provenant d'une station d'épuration des eaux usées, des produits de digestion de déchets organiques et des émulsifiants.

8. Procédé selon la revendication 7, **caractérisé en ce que** la quantité de composants supplémentaires dans la matière première susmentionnée contenant des composants organiques est de 10 à 50% en poids, de préférence de 20 à 40% en poids, par rapport au poids total de la matière première.

9. Procédé selon l'une quelconque des revendications précédentes, **caractérisé en ce qu'**aucune quantité supplémentaire d'eau n'est ajoutée à l'autoclave à l'étape i)

10. Procédé selon l'une quelconque des revendications précédentes, **caractérisé en ce qu'**une étape de séparation est réalisée sur le ou les plusieurs flux résiduel(s) obtenu(s) après l'étape iv) de manière à obtenir un flux résiduel riche en matière plastique et en suspension organique, où, de préférence, ladite suspension organique est en outre séparée en une fraction riche en fibres et une fraction aqueuse.

11. Procédé selon la revendication 10, **caractérisé en ce que** la suspension organique, en particulier la fraction aqueuse séparée de celle-ci, est amenée à une station de fermentation.

12. Procédé selon l'une quelconque des revendications précédentes, **caractérisé en ce que** l'étape iv) est réalisée après que la température de l'autoclave a diminué jusqu'au plus 110°C.

13. Procédé selon la revendication 10, **caractérisé en ce que** les matières plastiques sont séparées du flux résiduel susmentionné qui est riche en matières plastiques et retraitées en un flux de matière apte à être réutilisé.

14. Procédé selon l'une quelconque des revendications précédentes, **caractérisé en ce que** le ou les plusieurs flux résiduel(s) obtenu(s) après l'étape iv) contient/contiennent un pourcentage de principes actifs de médicaments qui est inférieur au pourcentage de principes actifs de médicaments de la matière première contenant des composants organiques qui a été fournie à l'autoclave dans l'étape i).

15. Utilisation d'un autoclave pourvu d'un mécanisme d'agitation pour effectuer le procédé selon l'une quelconque des revendications 1 à 14 pour traiter une matière première contenant des composants organiques qui comprend un(e) ou plusieurs parmi des couches et des matériels d'incontinence en un ou plusieurs flux résiduel(s), où le mécanisme d'agitation est pourvu d'un ou de plusieurs élément(s) qui tourne/tournent le long de la paroi interne de l'autoclave, ledit mécanisme d'agitation étant pourvu de bras qui sont longs et incurvés et/ou qui sont agencés selon un angle par rapport à l'horizontale, où l'espacement entre les extrémités desdits un ou plusieurs élément(s) et ladite paroi interne est d'au plus 10 cm.
